# EUROPÄISCHE PATENTSCHRIFT

(11) **EP 2 004 089 B1**
(45) Veröffentlichungstag und Bekanntmachung des Hinweises auf die Patenterteilung: **22.09.2010**
(21) Anmeldenummer: 07724040.6
(22) Anmeldetag: 05.04.2007
(51) Int. Cl.: A61F 2/00, A61F 5/00, A61M 25/00

(54) **VERSCHLUSSSYSTEM ZUR VERSORGUNG REKTALER BZW. ANALER INKONTINENZ**
OCCLUSION SYSTEM FOR MANAGEMENT OF RECTAL OR ANAL INCONTINENCE
SYSTÈME OBTURATEUR POUR LE TRAITEMENT DE L'INCONTINENCE RECTALE OU ANALE

(30) Priorität: 12.04.2006 DE 202006005951 U
(43) Veröffentlichungstag der Anmeldung: 24.12.2008
(73) Patentinhaber: BC & D Concepts GmbH, 69259 Wilhelmsfeld (DE)
(72) Erfinder: GÖBEL, Fred, 69259 Wilhelmsfeld (DE)
(74) Vertreter: Wagner, Matthias
(86) Internationale Anmeldenummer: PCT/EP2007/003099
(87) Internationale Veröffentlichungsnummer: WO 2007/118621

(56) Entgegenhaltungen:
- EP-A- 0 282 449
- EP-A- 1 535 588
- DE-A1-102004 033 425
- DE-C- 355 411
- JP-A- 10 234 854

## Beschreibung

Die Erfindung betrifft ein Verschlusssystem zur Versorgung rektaler bzw. analer Inkontinenz, umfassend ein Schaftelement mit einem distalen Ende und einem proximalen Ende sowie einem durchgehend vom distalen Ende zum proximalen Ende des Schaftelementes verlaufenden Lumen und einen haftfest und abgedichtet auf dem Schaftelement aufgebrachten Ballon, der über eine Füllleitung mit einem Füllmedium beaufschlagbar ist, wobei der Ballon aus einem Schlauchabschnitt gefertigt und derart vorgeformt ist, dass er bei Befüllung mit dem Füllmedium eine erste Ballonausformung als intrarektaler Ballon und eine zweite Ballonausformung als Widerlager-Ballon ausbildet, die mittels einer Einschnürung voneinander abgegrenzt sind.

Zur Versorgung von Patienten mit rektaler bzw. analer Inkontinenz sind bislang rektale Tamponaden aus quellfähigem, selbstentfaltendem Schaumstoff oder Zellulosematerial bekannt, die vom Patienten als gepresster, etwa zäpfchenförmiger Körper über den Anus in das Rektum eingeführt werden und dort durch Aufnahme von Darmsekret zum Teil um das Mehrfache ihres ursprünglichen Volumens aufquellen, wodurch Abdichtung gewährleistet wird. Allerdings sind derartige aufgequollene Tamponadenkörper nur schwer und für den Patienten in der Regel schmerzhaft zu entfernen, wobei darüber hinaus der prall im Rektum entfaltete Tamponadekörper vom Patienten häufig als irritierend empfunden wird und Stuhldranggefühl auslöst bzw. den Defäkationsreflex triggert. Ferner sind Quelltamponaden nicht reinigungsfähig und damit nicht wiederverwendbare Einmalartikel, was zu einem hohen Verbrauch an Tamponaden, bis zu 10 Stück pro Tag, führt.

Darüber hinaus sind auch Abdichtungselemente auf Basis aufblasbarer Ballons bekannt. So zeigen die gattungsbildenden DE 10 2004 033 425 A1 sowie JP 10 234854 A ein Verschlusssystem, bei welchem ein Schaftelement mit distalem Ende und proximalem Ende sowie durchgehenden Lumen innerhalb des Schaftelementes vorgesehen ist, welches mit dem distalen Ende voran über den Anus in das Rektum eingeführt wird. Auf dem Schaftelement ist haftfest und abgedichtet mindestens ein Ballon angeordnet, der über eine Füllleitung mit einem Füllmedium befüllbar ist, wobei der Ballon aus einem extrudierten Schlauchabschnitt einer Kunststofffolie gefertigt und derart vorgeformt ist, dass er bei Befüllung mit dem Füllmedium eine erste Ballonausformung als intrarektaler Ballon ausbildet, welcher die Abdichtung bewerkstelligt und darüber hinaus eine zweite Ballonausformung als Widerlager-Ballon ausbildet, welcher außerhalb des Anus zum Liegen kommt. Die beiden Ballonausformungen sind voneinander durch eine Einschnürung abgegrenzt, wobei sich die Vorformung des Ballons mit zwei Ballonausformungen und einer dazwischen befindlichen Einschnürung beispielsweise durch Umformung des Schlauchabschnittes im so genannten Hohlkörperblasverfahren oder auch Blow-Molding-Verfahren erreichen lässt.

Die Handhabung des bekannten Verschlusssystems lässt jedoch noch Raum für Verbesserungen und überdies gestaltet sich bislang die Ableitung von Darmgasen durch das Verschlusssystem als schwierig, da hierzu sowohl das distale wie auch das proximale Ende des Schaftelementes offen ausgebildet sein müssen, um die Durchleitung von Darmgas durch das Schaftelement des ansonsten den Darm abdichtenden Verschlusssystems zu gewährleisten. Hierbei kommt es jedoch sehr leicht zu einem Eintritt von Darminhalt in das Schaftelement, der sodann unerwünscht über das proximale Ende austritt oder aber einen Verschluss der Gaspassage innerhalb des durchgehenden Lumens des Schaftelementes hervorruft.

Aufgabe der vorliegenden Erfindung ist es daher, das gattungsgemäße Verschlusssystem dahingehend weiterzubilden, dass bei vereinfachter Handhabung desselben eine zuverlässige Ableitung von Darmgas aus dem Rektum ermöglicht wird, wobei gleichzeitig eine leichte Reinigung des Verschlusssystes die mehrfache Wiederverwendbarkeit für den Patienten gewährleisten soll.

Zur Lösung dieser gestellten Aufgabe wird erfindungsgemäße die Ausbildung eines Verschlusssystems zur Versorgung rektaler bzw. analer Inkontinenz gemäß den Merkmalen des Patentanspruches 1 vorgeschlagen.

Vorteilhafte Ausgestaltungen und Weiterbildungen des erfindungsgemäßen Verschlusssystems sind Gegenstand der Unteransprüche.

Erfindungsgemäß wird vorgesehen, dass das proximale Ende des Schaftelementes mit einem sich quer zur Längsachse des Schaftelementes erstreckendem Austrittsrohr mit endseitigen Austrittsöffnungen verbunden ist, welches seinerseits mit dem Lumen kommuniziert. Dieses sich vorzugsweise in einem 90°-Winkel zur Längsachse des Schaftelementes erstreckende Austrittsrohr dient bei Einführen und Herausziehen des erfindungsgemäßen Verschlusssystems als Handgriff und kommt in eingesetztem Zustand des Verschlusssystems in der Analfalte des Patienten zum Liegen, wo es nicht als störend empfunden wird. Gleichwohl ist über die beiden endseitigen Austrittsöffnungen eine zuverlässige Ableitung des über die mindestens eine Gaseintrittsöffnung in das Lumen des Schaftelementes eingetretenen Darmgases möglich, wobei aufgrund des abgewinkelten Verlaufs auch etwaige in das Schaftelement eingetretene Verunreinigungen zuverlässig von einem Austritt aus den Austrittsöffnungen abgehalten werden.

Darüber hinaus ist das proximale Ende des Ballons so ausgeformt, dass es sich bei Beaufschlagung mit dem Füllmedium schützend über das freie Ende des Ausschnittsrohres 110 legt, was den Tragekomfort für den Patienten erhöht.

Die im Bereich der Endkappe des erfindungsgemäßen Verschlusssystems vorgesehene mindestens eine Gaseintrittsöffnung ist nach einem Ausführungsbeispiel der Erfindung quer zur Längsachse des Schaftelementes in die Endkappe eingebracht.

Nach einer alternativen Ausführungsform des erfindungsgemäßen Verschlusssystems kann jedoch auch vorgesehen sein, dass die Endkappe das distale Ende des Schaftelementes pilzkopfartig übergreift und die mindestens eine Gaseinstrittsöffnung in dem das Schaftelement übergreifenden Bereich der Endkappe ausgebildet ist.

Um ein leichtes Einführen des erfindungsgemäßen Verschlusssystems zu gewährleisten und Verletzungen vorzubeugen, ist die Spitze der Endkappe bevorzugt atraumatisch ausgebildet.

Um die mehrfache Wiederverwendbarkeit des erfindungsgemäßen Verschlusssystems zu gewährleisten, muss eine leichte Reinigung desselben für einen Patienten möglich sein. Zu diesem Zweck ist bevorzugt das Austrittsrohr an einem, insbesondere aber an seinen beiden Enden mit entsprechenden Konnektoren für den Anschluss einer handelsüblichen Spritze ausgebildet, so dass die mit einer Reinigungsflüssigkeit aufgezogene Spritze an einem Ende des Austrittsrohres konnektiert werden kann und durch Betätigung der Spritze somit das gesamte gasführende System aus Austrittsrohr sowie Schaftelement mit Endkappe gespült und von daher auf einfachste Weise effektiv gereinigt werden kann.

Zur Sicherstellung einer guten Abdichtung des erfindungsgemäßen Verschlusssystems im Rektum sind bevorzugt die Enden des den Ballon ausbildenden Schlauchabschnittes nahe des proximalen Endes am Schaftelement abdichtend befestigt, beispielsweise auf dem Schaltelement verklebt oder verschweißt, so dass der Ballon in Richtung auf das distale Ende unter Ausbildung einer Einstülpung entlang des Schaftelementes verläuft.

Diese bereits aus der gattungsbildenden DE 10 2004 033 425 A1 bekannte Befestigung des Ballons unter Ausbildung einer Einstülpung hat zur Folge dass der Ballon, hier der intrarektale Ballon, bei Befüllung mit dem Füllmedium eine Rollbewegung entlang der Längsachse des Schaftelementes ausführt, und zwar in Richtung auf das proximale Ende desselben, die demgemäß bei in das Rektum eingeführtem intrarektalen Ballon in Richtung auf den Anus gerichtet ist und von daher eine zuverlässige Abdichtung des Rektums nach außen sicherstellt. Wegen weiterer Einzelheiten dieser Rollbewegung wird auf die bereits erwähnte DE 10 2004 033 425 A1 verwiesen.

Gemäß einer alternativen Ausgestaltung des erfindungsgemäßen Verschlusssystems ist ein Ende des den Ballon ausbildenden Schlauchabschnittes nahe des distalen Endes am Schaftelement befestigt und das andere Ende des den Ballon ausbildenden Schlauchabschnittes nahe des proximalen Endes am Schaftelement befestigt. Besonders bevorzugt ist es hierbei, dass die jeweiligen Enden des den Ballon ausbildenden Schlauchabschnittes unter Ausbildung einer Taschenfalte am Schaftelement befestigt sind, die vorzugsweise zur Aussenseite des Ballons 2 hin geöffnet ist. Aufgrund dieser Ausbildung einer Taschenfalte am distalen und proximalen Ende des den Ballon ausbildenden Schlauchabschnittes führen das distale und proximale Ende des Ballons bei Beaufschlagung mit dem Füllmedium jeweils eine entgegengesetzt gerichtete Rollbewegung in Richtung auf die Einschnürung entlang der Längsachse des Schaftelementes aus, so dass sich in eingeführtem Zustand des Verschlusssystems der intrarektale Ballon abdichtend auf die Innenseite des Anus legt und eine zuverlässige Abdichtung bewirkt, während andererseits der Widerlager-Ballon sich abdichtend auf die Außenseite des Anus legt und für einen festen Sitz des Verschlusselementes sorgt. Dieser feste Sitz mit zuverlässiger Abdichtung wird darüber hinaus besonders gefördert, wenn die Summe der axialen Rollbewegungen des distalen und proximalen Endes des Ballons bzw. der sich dort ausbildenden intrarektalen bzw. Widerlager-Ballon mindestens der axialen Länge der Einschnürung entspricht oder größer als diese ist. Unter axialer Länge der Einschnürung wird der Längenabschnitt der Einschnürung in Richtung der Längsachse des Schaftelementes verstanden.

Der Ballon des erfindungsgemäßen Verschlusssystems ist bevorzugt aus einem Schlauchabschnitt auf Basis einer Polyurethanfolie mit einer Wanddicke von 5 bis 45 µm hergestellt. Es können auch mehrlagige, miteinander verschweißte Folienlagen, z.B. aus Polyethylen, Polyvinylchlorid oder Polyurethan zur Ausbildung des Schlauchabschnittes zum Einsatz kommen. Auch ist die Herstellung des Schlauchabschnittes aus Silikon oder Naturlatex im Tauchverfahren möglich.

Nach einem Vorschlag der Erfindung kann die Endkappe als separates Bauteil auf das distale Ende des Schaftelementes aufgesteckt und dort mit geeigneten Mitteln dauerhaft fixiert werden, beispielsweise eingeklebt oder verschweißt werden.

Nach einem weiteren Vorschlag der Erfindung ist es jedoch auch denkbar, die Endkappe integral am distalen Ende des Schaftelementes anzuformen.

Bei Bedarf kann ferner innerhalb des durchgehenden Lumens des Schaftelementes eine Adsorbermasse vorgesehen sein, um das Darmgas zu filtern. Hierzu kommt insbesondere eine Aktivkohle in Betracht.

Darüber hinaus kann die Füllleitung mit einem Ventil und einem Pilotballon zum Ertasten des Fülldruckes des Ballons ausgebildet sein, so dass die Befüllung des Ballons wie auch dessen Entleerung über eine an die Füllleitung angeschlossene Spritze erfolgen kann.

Wesentlicher Vorteil des erfindungsgemäßen Verschlusssystems ist nicht nur seine durch die Entfaltung der beiden Ballonausformungen hervorgerufene zuverlässige Abdichtung, sondern auch die leichte Einführung bzw. das leichte Herausziehen des Verschlusssystems aus dem Anus, wenn zuvor das Füllmedium aus den Ballons über die an die Füllleitung angeschlossene Spritze abgesaugt worden ist.

Um einen besonders angenehmen Tragekomfort für den Patienten zu gewährleisten, weist das Schaftelement einen möglichst kleinen Außendurchmesser auf, beispielsweise einen Außendurchmesser von etwa 4 bis 8 mm und die den intrarektalen Ballon ausbildende Ballonausformung weist im befüllten aber nicht gedehnten Zustand einen Durchmesser von etwa 30 bis 80 mm und eine Länge von bis zu 20 cm auf.

Bei Ausbildung eines im Hinblick auf den Tragekomfort vorzugsweise dünn ausgebildeten Schaftelementes kann dasselbe im Verbindungsbereich zum Bal-Ion mit einer Verdickung ausgebildet sein, um ausreichend Befestigungsfläche für den Ballon bzw. die Enden des den Ballon ausbildenden Schlauchabschnittes zu schaffen.

Weitere Ausgestaltungen und Einzelheiten des erfindungsgemäßen Verschlusssystems werden nachfolgend anhand von Ausführungsbeispielen in der Zeichnung erläutert. Es zeigen:
- Figur 1: in schematisierter Darstellung ein Verschlusssystem in einer ers- ten Ausführungsform der Erfindung,
- Figur 2: in vergrößerter Darstellung die Endkappe des Verschlusssystems gemäß einer zweiten Ausführungsform der Erfindung,
- Figur 3: eine weitere Ausführungsform der Erfindung.

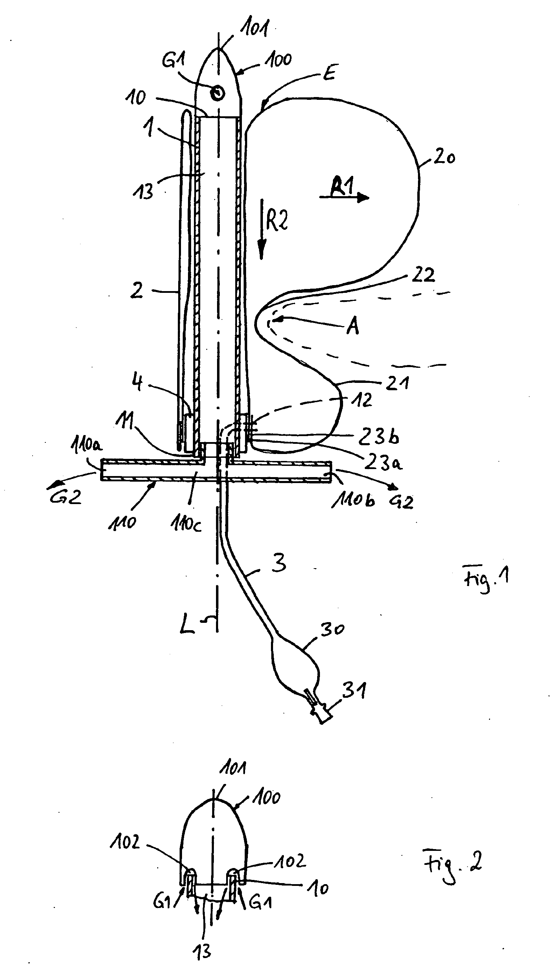

In der Figur 1 ist ein Verschlusssystem zur Versorgung rektaler bzw. analer Inkontinenz dargestellt, welches ein zentrales Schaftelement 1 beispielsweise aus einem elastisch verformbaren Kunststoffrohr mit einem distalen Ende 10 und einem proximalen Ende 11 sowie einem vom distalen Ende 10 bis zum proximalen Ende 11 durchgängigen und an beiden Enden offenen Lumen 13 aufweist.

Auf dem Schaftelement ist ein aus einem Schlauchabschnitt auf Basis einer Polyurethanfolie mit einer Wanddicke von beispielsweise 15 µm ausgebildeter Ballon 1 in nachfolgend noch näher beschriebener Weise befestigt, der über eine Füllleitung 3 und eine entsprechende Perforation 12 mit einem Füllmedium zur Entfaltung desselben befüllbar ist.

Ferner ist das distale Ende 10 des Schaftelementes 1 mit einer Endkappe 100 ausgestattet, während am proximalen Ende 11 des Schaftelementes 1 ein quer zur Längsachse L des Schaftelementes 1 verlaufendes Austrittsrohr 110 vorgesehen ist.

Aus der schematischen Schnittdarstellung gemäß Figur 1 ist der am Schaftelement 1 befestigte Ballon 2 in zwei voneinander abweichenden Zuständen sichtbar, nämlich rechts der Mittelachse L in seinem entfalteten, d.h. mit Füllmedium beaufschlagtem und links von der Mittelachse L in seinem eingefalteten, d.h. nicht mit Füllmittel beaufschlagten Zustand dargestellt.

Zur Bewirkung eines Verschlusses des Rektums bei rektaler bzw. analer Inkontinenz wird das Verschlusssystem mit der am distalen Ende 10 des Schaftelementes 1 befestigten Endkappe 100 voran und mit in eingefaltetem Zustand vorliegenden Ballon 2 durch den Anus A in das Rektum des Patienten eingeführt, wobei das Verschlusssystem vom Patienten an dem im Bereich des proximalen Endes 11 vorgesehenen Austrittsrohr 110 bequem gehalten werden kann. Das Austrittsrohr 110 kommt in der Endposition im Bereich der Analfalte des Patienten zum Liegen, wo es kaum spürbar ist.

Um beim Einführen des Verschlusssystems Verletzungen vorzubeugen und die Aufweitung des Anus A leicht zu bewerkstelligen, ist die Endkappe 100 mit einer atraumatischen Spitze 101 versehen.

Nach dem Einführen des Verschlusssystems kann sodann ein Füllmedium, beispielsweise Luft über die Füllleitung 3 in den Ballon 2 eingebracht werden, wozu am Ende der Füllleitung 3 ein entsprechender Konnektor 31 mit einem Ventil vorgesehen ist, so dass eine Luft enthaltende aufgezogene Spritze hier konnektiert werden und mittels der Spritze Luft über die Füllleitung 3 in das Innere des Ballons 2 eingeleitet werden kann. Der sich dabei innerhalb des Ballons 2 einstellende Fülldruck kann vom Patienten anhand des Pilotballons 30 an der Füllleitung 3 ertastet werden.

Der Ballon 2 ist aus einem flexiblen Schlauchabschnitt einer Kunststofffolie beispielsweise auf Basis von Polyurethan gefertigt, wobei dessen beide Schlauchenden 23a, 23b gemeinsam nahe des proximalen Endes 11 am Schaftelement 1 abdichtend befestigt sind und wobei die Perforation 12 zur Einleitung des Füllmediums in das Innere des Ballons 2 einmündet.

Wenn das Schaftelement 1 zur Erhöhung des Tragekomforts mit einem geringen Außendurchmesser von beispielsweise 4 bis 8 mm ausgeführt ist, kann zur Schaffung einer ausreichenden Befestigungsfläche für die Enden 23a, 23b des Ballons in diesem Befestigungsbereich nahe des proximalen Endes 11 ein verdicktes Endstück 4 vorgesehen sein, auf welchem die beiden Enden 23a, 23b des Schlauchabschnittes befestigt, beispielsweise aufgeklebt oder verschweißt werden.

Durch die zuvor erwähnte Befestigung sowohl des proximalen Endes 23a des Schlauchabschnittes wie auch des distalen Endes 23b des Schlauchabschnittes zur Ausformung des Ballons 2 wird der ausgebildete Ballon 2 in Richtung auf das distale Ende 10 des Schaftelementes unter Ausbildung einer Einstülpung E am Schaftelement 1 anliegend, jedoch mit diesem außerhalb des verdickten Endstückes 4 nicht fixiert geführt, so dass er weitgehend unabhängig vom Schaftelement 1 beweglich ist und sich an die Anatomie des Patienten anpassen kann.

Darüber hinaus ist der den Ballon 2 ausbildende Schlauchabschnitt durch eine bei der Herstellung bewirkte Ausformung z.B. im so genannten Hohlkörperblasverfahren bzw. Blow-Molding-Verfahren derart vorgeformt, dass er bei Befüllung mit dem Füllmedium 2 durch eine Einschnürung 22 voneinander abgeteilte Ballonausformungen 20, 21 ausbildet, von denen die Ballonausformung 20 den intrarektalen Ballon bildet und bei Anwendung des Verschlusssystems sich innerhalb des Rektums des Patienten entfaltet, während die Ballonausformung 21 als Widerlager-Ballon außerhalb des Anus A des Patienten zum Liegen kommt und das Verschlusssystem stabilisiert.

Aufgrund der zuvor bereits beschriebenen Befestigung des Ballons 2 unter Ausbildung einer Einschnürung E am Schaftelement 1 führt darüber hinaus die den intrarektalen Ballon ausbildende Ballonausformung 20 bei Befüllung mit einem Füllmedium und entsprechender Expansion in Pfeilrichtung A gleichzeitig auch eine entlang der Längsachse L des Schaftelementes in Richtung auf das proximale Ende 11 derselben gerichtete Rollbewegung gemäß Pfeil R aus, so dass eine zuverlässige und hoch abdichtende Anlage am Anus A des Patienten bewirkt wird, und von daher das Rektum zuverlässig verschlossen wird.

Um in dieser rechts der Mittellinie L in Figur 1 dargestellten Verschlussposition des Verschlusssystems die Ableitung von Darmgasen zu ermöglichen, ist die Endkappe 100 des Schaftelementes 1 mit mindestens einer Gaseintrittsöffnung 102 versehen, die über das distale Ende des Schaftelementes 1 mit dem durchgehenden Lumen 13 desselben kommuniziert.

Andererseits kommuniziert das durchgehende Lumen 13 im Bereich des proximalen Endes 11 mit dem im Innern des Austrittsrohres 110 ausgebildeten durchgehenden Lumens 110c, so dass es Darmgas ermöglicht ist, über die Gaseintrittsöffnung 102 gemäß Pfeil G1 in das durchgehende Lumen 13 des Schaftelementes 1 einzutreten und von dort über die beiden Austrittsöffnungen 110a, 110b des Austrittsrohres 110 gemäß Pfeilen G2 zu entweichen.

Wesentliches Merkmal der im Bereich der Endkappe 1 ausgebildeten mindestens einen Gaseintrittsöffnung 102 ist es, dass diese beabstandet von der Spitze 101 der Endkappe 10 angeordnet ist, hier quer zur Längsachse L des Schaftelementes. Aufgrund dieser beabstandeten und quer zur Längsachse L des Schaftelementes 1 vorgesehenen Anordnung kann die mindestens eine Gaseintrittsöffnung 102 auch bei auf der Endkappe 100 lastender Stuhlsäule nicht von Darminhalt verschlossen werden, so dass eine dauerhafte zuverlässige Gasabfuhr gewährleistet ist.

Sollte es dennoch zu einem Übertritt von Darmsekret über die Gaseinstrittsöffnung 102 in das Lumen 13 des Schaftelementes 1 kommen, so wird dieses aufgrund des zum proximalen Ende 11 hin rechtwinkligen Übergangs in das Austrittsrohr 110 zuverlässig an einem Austritt über die Austrittsöffnungen 110a, 110b gehindert.

Soll das vorangehend erläuterte Verschlusssystem wieder aus dem Rektum entfernt werden, so wird in umgekehrter Weise das Füllmedium aus dem Innern des Ballons 2 über die Füllleitung 3 abgesaugt, so dass sich wieder der eingefaltete Zustand gemäß der Darstellung links der Längsachse L des Schaftelementes 1 einstellt und das Verschlusssystem ohne großen Widerstand aus dem Anus A herausgezogen werden kann. Nachfolgend kann das gesamte Verschlusssystem durch Ansetzen einer mit Reinigungsflüssigkeit befüllten Spritze an eine der Austrittsöffnungen 110a, 110b des Austrittsrohres 110 gespült werden. Zu diesem Zweck sind die beiden Austrittsöffnungen 110a, 110b in Form eines handelsüblichen Konnektors gestaltet, so dass eine entsprechende Spritzenkonnektierung erfolgen kann.

Aufgrund dieser leichten Spülbarkeit und Reinigung des gesamten Verschlusssystems ist dieses zur mehrmaligen Verwendung durch den Patienten geeignet.

In einer in der Figur 2 dargestellten alternativen Ausführungsform der Endkappe 100 des Verschlusssystems sind die Gaseinstrittsöffnungen 102 nicht quer zur Längsachse L des Schaftelementes 1 vorgesehenen, sondern die Endkappe 100 übergreift das distale Ende 10 des Schaftelementes 1 pilzkopfartig, so dass die Endkappe 100 einen gewissen radialen Überstand aufweist, und in diesem übergreifenden Bereich der Endkappe 100 können sodann die Gaseintrittsöffnungen 102 ausgebildet werden, so dass es wiederum Gasen ermöglicht ist, gemäß Pfeilen G1 in das durchgehende Lumen 13 des Schaftelementes 1 überzutreten.

Auch bei dieser Ausführungsform wird zuverlässig der Verschluss der Gaseintrittsöffnungen 102 durch Darminhalt verhindert.

In jedem der vorangehend erläuterten Ausführungsbeispiele kann die eingesetzte Endkappe 100 als separates Teil zum Schaftelement 1 gefertigt werden und haftfest am distalen Ende 10 desselben befestigt werden, beispielsweise angeklebt oder verschweißt werden.

Allerdings ist es auch denkbar, eine derartige Endkappe integral an das distale Ende 10 des Schaftelementes 1 anzuformen.

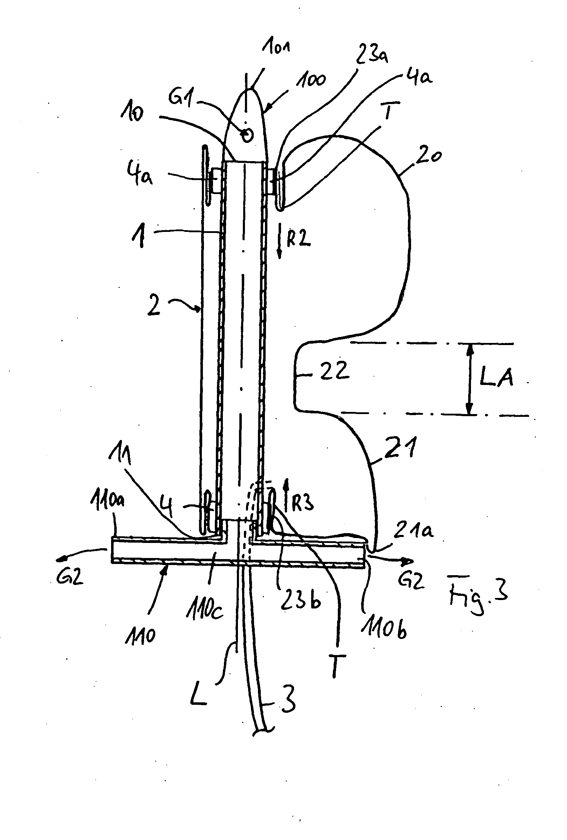

In der Figur 3 ist eine abgewandelte Ausführungsform des vorangehend erläuterten Verschlusssystems dargestellt, bei dem gleiche Teile gleiche Bezugsziffern erhalten und zur Vermeidung von Wiederholungen nicht nochmals gesondert erläutert werden, sofern dies nicht für das Verständnis der Ausführungsform erforderlich ist.

Der wesentliche Unterschied zwischen der Ausführungsform gemäß Figur 1 und Figur 3 liegt in der Befestigung des den intrarektalen Ballon 20 und den Widerlager-Ballon 21 ausbildenden Schlauchabschnittes 2, der wiederum auf Basis einer Polyurethanfolie oder auf einer verschweißten mehrlagigen Folie oder auch einer im Tauchverfahren hergestellten Silikon- oder Naturlatexmasse gebildet sein kann.

Die Befestigung des Schlauchabschnittes 2 am Schaftelement 1 erfolgt derart, dass das dem intrarektalen Ballon 20 benachbarte distale Ende 23a des Schlauchabschnittes 2 nahe dem distalen Ende am Schaftelement 1 über Verdickungen 4a befestigt ist, während das dem Widerlager-Ballon 21 benachbarte proximale Ende 23b des Schlauchabschnittes 2 über Verdickungen 4 nahe dem proximalen Ende 11 des Schlauchabschnittes 1 an diesem befestigt ist.

Die Befestigung der distalen bzw. proximalen Enden 23a, b des Schlauchabschnittes 2 am Schaftelement F1 erfolgt in der Weise, dass sich jeweils eine zur Außenseite des Schlauchabschnittes 2 offene Taschenfalte T am jeweiligen Befestigungspunkt zum Schaftelement 1 ausbildet. Diese Taschenfalte T ist dafür verantwortlich, dass die distalen bzw. proximalen Enden 23a, b des Schlauchabschnittes 2 bzw. der sich bei Befüllung mit einem Füllmedium ausbildende intrarektale Ballon 20 und der Widerlager-Ballon 21 gemäß Pfeilen R2, R3 eine entgegengesetzt aufeinander zu gerichtete Rollbewegung in Richtung auf die Einschnürung 22 ausführen, und zwar in axialer Richtung entlang der Längsachse L des Schaftelementes 1. Diese Rollbewegungen gemäß Pfeilen R2, R3 haben zur Folge, dass sich der ausbildende intrarektale Ballon 20 von innen an den an der Einschnürung 22 in Gebrauchsposition des Verschlusssystems befindlichen Anus abdichtend anlegt, während der Widerlager-Ballon 21 sich von außen an den Anus anlegt, um eine sichere Fixierung des Verschlusselementes zu bewirken. Die Größe der Taschenfalten T bestimmt hierbei die mögliche Rollbewegung in Pfeilrichtung R2 bzw. R3 und kann vom Fachmann entsprechend dem Bedarf festgelegt werden. Von besonderem Vorteil ist es jedoch, wenn die Summe der axialen Rollbewegungen R2, R3 mindestens gleich groß oder größer als die in Richtung der Längsachse L verlaufende axiale Länge LA der Einschnürung 22 ist.

Der Darstellung gemäß Figur 3 ist weiterhin entnehmbar, dass der Widerlager-Ballon 21 mit einer Ausformung 21a versehen ist, die sich bei Enthaltung des Widerlager-Ballons 21 über das freie Ende des Austrittsrohres 110 legt, um den Tragekomfort für den Patienten zu erhöhen und das Austrittsrohr nach Art eines luftbefüllten Kissens aufzunehmen.

Neben der vorangehend erläuterten Funktion des Verschlusssystems zur Versorgung rektaler bzw. analer Inkontinenz eignet sich dieses auch zur vorübergehenden Versorgung von Hämorrhoidalblutungen, da mittels des Ballons 2 eine für den Patienten angenehme blutstillende und den Zutritt von Darminhalt zuverlässig verhindernde Druckbeaufschlagung bewirkt werden kann.

Darüber hinaus eignet sich das vorangehend erläuterte Verschlusssystem auch zur Versorgung von End-zu-End-Anastomosen bei anusnahen Colonresektionen, indem die Operationsnaht oder der verwendete Clips vor Stuhl und Sekret des Patienten über mehrere Tage geschützt wird. Ferner kann auch die Entlastung von Darmgasen während Radiotherapieanwendungen, z.B. Seed-Bestrahlungen der Prostata in Kombination mit einem rektal platzierten Gewebeverdrängungsballon erfolgen.

## Patentansprüche

1. Verschlusssystem zur Versorgung rektaler bzw. analer Inkontinenz, umfassend ein Schaftelement (1) mit einem distalen Ende (10) und einem proximalen Ende (11) sowie einem durchgehend vom distalen Ende (10) zum proximalen Ende (11) des Schaftelementes (1) verlaufenden Lumen (13) und einen haftfest und abgedichtet auf dem Schaftelement (1) aufgebrachten Ballon (2), der über eine Füllleitung (3) mit einem Füllmedium beaufschlagbar ist, wobei der Ballon (2) aus einem Schlauchabschnitt gefertigt und derart vorgeformt ist, dass er bei Befüllung mit dem Füllmedium eine erste Ballonausformung (20) als intrarektaler Ballon und eine zweite Ballonausformung (21) als Widerlager-Ballon ausbildet, die mittels einer Einschnürung (22) voneinander abgegrenzt sind, wobei das distale Ende (10) des Schaftelementes (1) mit einer Endkappe (100) mit einer Spitze (101) ausgebildet ist und beabstandet von der Spitze (101) mindestens eine mit dem Lumen (13) des Schaftelementes (1) kommunizierende Gaseintrittsöffnung (102) vorgesehen ist, und das proximale Ende (11) des Schaftelementes (1) mit einem Austrittsrohr (110) verbunden ist, welches mit dem Lumen (13) kommuniziert, **dadurch gekennzeichnet, dass** das Austrittsrohr (110) sich quer zur Längsachse (L) des Schaftelementes (1) erstreckt und endseitige Austrittsöffnungen (110a, b) aufweist und das proximale Ende des Ballons so ausgeformt ist, dass es sich bei Beaufschlagung mit dem Füllmedium über das freie Ende des Austrittsrohres (110) legt.

2. Verschlusssystem nach Anspruch 1, **dadurch gekennzeichnet, dass** die mindestens eine Gaseintrittsöffnung (102) quer zur Längsachse (L) des Schaftelementes (1) in die Endkappe (1) eingebracht ist.

3. Verschlusssystem nach Anspruch 1, **dadurch gekennzeichnet, dass** die Endkappe (1) das distale Ende (10) des Schaftelementes (1) pilzkopfartig übergreift und die mindestens eine Gaseintrittsöffnung (102) in dem das Schaftelement (1) übergreifenden Bereich der Endkappe (100) ausgebildet ist.

4. Verschlusssystem nach einem der Ansprüche 1 bis 3, **dadurch gekennzeichnet, dass** die Spitze (101) der Endkappe (100) atraumatisch ausgebildet ist.

5. Verschlusssystem nach einem der Ansprüche 1 bis 4, **dadurch gekennzeichnet, dass** das Austrittsrohr (100) an seinen beiden Enden (110a, 110b) mit Konnektoren für den Anschluss einer Spritze ausgebildet ist.

6. Verschlusssystem nach einem der Ansprüche 1 bis 5, **dadurch gekennzeichnet, dass** der den Ballon (2) ausbildende Schlauchabschnitt aus einer mindestens einlagigen Kunststofffolie gebildet ist.

7. Verschlusssystem nach einem der Ansprüche 1 bis 6, **dadurch gekennzeichnet, dass** der Ballon (2) aus einem Schlauchabschnitt auf Basis einer Polyurethanfolie mit einer Wanddicke von 5 bis 45 µm hergestellt ist.

8. Verschlusssystem nach einem der Ansprüche 1 bis 7, **dadurch gekennzeichnet, dass** die Enden (23a, 23b) des den Ballon (2) ausbildenden Schlauchabschnittes nahe des proximalen Endes (11) am Schaftelement (1) abdichtend befestigt sind, so dass der Ballon (2) in Richtung auf das distale Ende (10) unter Ausbildung einer Einstülpung entlang des Schaftelementes verläuft.

9. Verschlusssystem nach einem der Ansprüche 1 bis 5, **dadurch gekennzeichnet, dass** der den Ballon (2) ausbildende Schlauchabschnitt auf Basis von Silikon oder Naturlatex im Tauchverfahren hergestellt ist.

10. Verschlusssystem nach einem der Ansprüche 1 bis 7, **dadurch gekennzeichnet, dass** ein Ende (23a) des den Ballon (2) ausbildenden Schlauchabschnittes nahe des distalen Endes 810) am Schaftelement (1) befestigt ist und das andere Ende (23b) des den Ballon (2) ausbildenden Schlauchabschnittes nahe des proximalen Endes (11) am Schaftelement (1) befestigt ist.

11. Verschlusssystem nach Anspruch 10, **dadurch gekennzeichnet, dass** die Enden (23a, 23b) des den Ballon (2) ausbildenden Schlauchabschnittes unter Ausbildung einer Taschenfalte (T) am Schaftelement (1) befestigt sind, so dass das distale und proximale Ende des Ballons (2) bei Beaufschlagung mit dem Füllmedium eine Rollbewegung (R2, R3) in Richtung auf die Einschnürung (22) entlang der Längsachse (L) des Schaftelementes (1) ausführen und die Summe der axialen Rollbewegungen (R2, R3) mindestens der axialen Länge (LA) der Einschnürung (22) entspricht oder größer als diese ist.

12. Verschlusssystem nach einem der Ansprüche 1 bis 11, **dadurch gekennzeichnet, dass** die Endkappe (100) integral am distalen Ende (10) des Schaftelementes (1) angeformt ist.

13. Verschlusssystem nach einem der Ansprüche 1 bis 12, **dadurch gekennzeichnet, dass** innerhalb des durchgehenden Lumens (13) des Schaftelementes (1) eine Adsorbermasse vorgesehen ist.

14. Verschlusssystem nach einem der Ansprüche 1 bis 13, **dadurch gekennzeichnet, dass** die Füllleitung (3) mit einem Ventil und einem Pilotballon (30) zum Ertasten des Fülldruckes des Ballons (2) ausgebildet ist.

15. Verschlusssystem nach einem der Ansprüche 1 bis 14, **dadurch gekennzeichnet, dass** das Schaftelement (1) einen Außendurchmesser von 4 bis 8 mm aufweist und die den intrarektalen Ballon ausbildende Ballonausformung (20) im befüllten, nicht gedehnten Zustand einen Durchmesser von 30 bis 80 mm und eine Länge von 40 mm bis 20 cm aufweist.

16. Verschlusssystem nach einem der Ansprüche 1 bis 15, **dadurch gekennzeichnet, dass** das Schaftelement 1 im Verbindungsbereich zum Ballon 2 verdickt ausgebildet ist.

## Claims

1. Occlusion system for managing rectal or anal incontinence, comprising a shaft element (1) with a distal end (10) and a proximal end (11) as well as a lumen running continuously from the distal end (10) to the proximal end (11) of the shaft element (1) and a balloon (2), which is attached in a fixed and sealed manner to the shaft element (1) and can be charged via a filling line (3) with a filling medium, the balloon (2) being manufactured from a hose portion and being preformed in such a way that, on filling with the filling medium, it forms a first balloon formation (20) as an intra-rectal balloon and a second balloon formation (21) as a support balloon, which are delimited with respect to one another by means of a constriction (22), the distal end (10) of the shaft element (1) being configured with an end cap (100) with a tip (101) and, at a spacing from the tip (101), at least one gas inlet opening (102) communicating with the lumen (13) of the shaft element (1) being provided, and the proximal end (11) of the shaft element being connected to an outlet tube (110), which communicates with the lumen (13), **characterised in that** the outlet tube (110) extends transverse to the longitudinal axis (L) of the shaft element (1) and has end outlet openings (110a, b) and the proximal end of the balloon is formed in such a way that on charging with the filling medium, it is placed over the free end of the outlet tube (110).

2. Occlusion system according to claim 1, **characterised in that** the at least one gas inlet opening (102) is introduced into the end cap (1) transverse to the longitudinal axis (L) of the shaft element (1).

3. Occlusion system according to claim 1, **characterised in that** the end cap (1) engages in a mushroom cap-shape over the distal end (10) of the shaft element (1) and the at least one gas inlet opening (102) is configured in the region of the end cap (100) engaging over the shaft element (1).

4. Occlusion system according to any one of claims 1 to 3, **characterised in that** the tip (101) of the end cap (100) is atraumatically configured.

5. Occlusion system according to any one of claims 1 to 4, **characterised in that** the outlet tube (100), at its two ends (110a, 110b), is configured with connectors to connect a syringe.

6. Occlusion system according to any one of claims 1 to 5, **characterised in that** the hose portion forming the balloon (2) is formed from an at least one-layered plastics material film.

7. Occlusion system according to any one of claims 1 to 6, **characterised in that** the balloon (2) is produced from a hose portion based on a polyurethane film with a wall thickness of 5 to 45 µm.

8. Occlusion system according to any one of claims 1 to 7, **characterised in that** the ends (23a, 23b) of the hose portion forming the balloon (2) are fastened in a sealing manner close to the proximal end (11) to the shaft element (1), so the balloon (2) extends along the shaft element in the direction of the distal end (10) with the formation of an inversion.

9. Occlusion system according to any one of claims 1 to 5, **characterised in that** the hose portion forming the balloon (2) is produced on the basis of silicone or natural latex by the dipping method.

10. Occlusion system according to any one of claims 1 to 7, **characterised in that** one end (23a) of the hose portion forming the balloon (2) is fastened close to the distal end (10) to the shaft element (1) and the other end (23b) of the hose portion forming the balloon (2) is fastened close to the proximal end (11) to the shaft element (1).

11. Occlusion system according to claim 10, **characterised in that** the ends (23a, 23b) of the hose portion forming the balloon (2) are fastened, with the formation of a pocket fold (T), on the shaft element (1), so the distal and proximal end of the balloon (2), when charged with the filling medium, carry out a rolling movement (R2, R3) in the direction of the constriction (22) along the longitudinal axis (L) of the shaft element (1) and the sum of the axial rolling movements (R2, R3) corresponds at least to the axial length (LA) of the constriction (22) or is larger than this.

12. Occlusion system according to any one of claims 1 to 11, **characterised in that** the end cap (100) is formed integrally on the distal end (10) of the shaft element (1).

13. Occlusion system according to any one of claims 1 to 12, **characterised in that** an adsorber mass is provided within the continuous lumen (13) of the shaft element (1).

14. Occlusion system according to any one of claims 1 to 13, **characterised in that** the filling line (3) is configured with a valve and a pilot balloon (30) to detect the filling pressure of the balloon (2).

15. Occlusion system according to any one of claims 1 to 14, **characterised in that** the shaft element (1) has an external diameter of 4 to 8 mm and the balloon formation (20) forming the intra-rectal balloon, in the filled, non-extended state, has a diameter of 30 to 80mm and a length of 40 mm to 20 cm.

16. Occlusion system according to any one of claims 1 to 15, **characterised in that** the shaft element (1) is configured with a thickening in the connecting region to the balloon (2).

## Revendications

1. Système obturateur pour le traitement de l'incontinence rectale ou anale, comprenant un élément de tige (1) avec une extrémité distale (10) et une extrémité proximale (11) ainsi qu'une lumière (13) qui s'étend d'un bout à l'autre de l'extrémité distale (10) à l'extrémité proximale (11) de l'élément de tige (1) et un ballonnet (2), placé de manière solidaire et étanche sur l'élément de tige (1), qui peut être rempli par le biais d'une conduite de remplissage (3) avec un fluide de remplissage, le ballonnet (2) étant constitué d'un segment de tube et préformé de façon à réaliser lors du remplissage avec le fluide de remplissage une première déformation du ballonnet (20) en tant que ballonnet intrarectal et une deuxième déformation du ballonnet (21) en tant que ballonnet de contre-appui qui sont séparés l'une de l'autre par une gorge (22) et l'extrémité distale (10) de l'élément de tige (1) étant réalisée avec un capuchon d'extrémité (100) avec une pointe (101) et, à distance de la pointe (101), il est prévu au moins un orifice d'arrivée de gaz (102) communiquant avec la lumière (13) de l'élément de tige (1) et l'extrémité proximale (11) de l'élément de tige (1) est reliée à un tube de sortie (110) qui communique avec la lumière (13), **caractérisé en ce que** le tube de sortie (110) s'étand transversalment à l'axe longitudinale (L) de l'élément de tige (1) et présente des orifices de sortie terminaux (110a, 110b) et l'extrémité proximale du ballonet (2) est formée de telle sorte que lors du remplissage avec le fluide de remplissage il gît sur l'extrémité libre du tube de sortie (110).

2. Système obturateur selon la revendication 1, **caractérisé en ce que** le au moins un orifice d'entrée de gaz (102) est emmené, trasversalement par rapport à l'axe longitudinal de l'élément de tige (1), dans le capuchon d'extrémité (1).

3. Système obturateur selon la revendication 1, **caractérisé en ce que** le capuchon d'extrémité (1) recouvre en forme de tête de champignon l'extrémité distale (10) de l'élément de tige (1) et le au moins un orifice d'entrée de gaz (102), dans lequel le secteur du capuchon d'extrémité (100) vient en prise avec l'élément de tige (1), est formé.

4. Système obturateur selon une des revendications de 1 à 3, **caractérisé en ce que** la pointe (101) du capuchon d'extrémité (100) est formée de sorte à ne pas provoquer des traumatismes.

5. Système obturateur selon une des revendications de 1 à 4, **caractérisé en ce que** le tube de sortie (100) est formé au niveau de ses deux extrémités (110a, 100b) avec des connecteurs pour le branchement d'une seringue.

6. Système obturateur selon une des revendications de 1 à 5, **caractérisé en ce que** le segment de tube formant le ballonnet (2) est constitué à partir d'une feuille en matière synthétique au moins à une couche.

7. Système obturateur selon une des revendications de 1 à 6, **caractérisé en ce que** le ballonnet (2) est produit à partir d'un segment de tube sur la base d'une feuille en polyuréthane avec une épaisseur de paroi allant de 5 à 45 µm.

8. Système obturateur selon une des revendications de 1 à 7, **caractérisé en ce que** les extrémités (23a, 23b) du segment de tube formant le ballonnet (2) sont rattachées de manière étanche à proximité de l'extrémité proximale (11) à l'élément de tige (1), de sorte que le ballonnet (2) passe, en direction de l'étrémité distale (10), avec la formation d'un retournement, le long de l'élément de tige (1).

9. Système obturateur selon une des revendications de 1 à 5, **caractérisé en ce que** le segment de tube formant le ballonnet (2) est produit par trempage à base de silicone ou latex naturel.

10. Système obturateur selon une des revendications de 1 à 7, **caractérisé en ce qu'**une extrémité (23a) du segment de tube formant le ballonnet (2) est rattachée à proximité de l'extrémité distale (10) à l'élément de tige (1) et que l'autre extrémité (23b) du segment de tube formant le ballonnet (2) est rattachée à proximité de l'extrémité proximale (11) à l'élément de tige (1).

11. Système obturateur selon la revendication 10, **caractérisé en ce que** les extrémités (23a, 23b) du segment de tube formant le ballonet (2) sont rattachées à l'élément de tige (1) par la formation d'un repli de poche (T), de sorte que les extrémités distale et proximale du ballonet (2), lors du remplissage avec le fluide de remplissage effectuent un mouvement de rotation (R2, R3) en direction de la gorge (22) le long de l'axe longitudinal (L) de l'élément de tige (1) et la somme des mouvements de rotation (R2, R3) axiaux correspond au moins à la longeur axiale (LA) de la gorge (22) ou est plus grande que celle-ci.

12. Système obturateur selon une des revendications de 1 à 11, **caractérisé en ce que** le capuchon d'extrémité (100) est formé, d'une seule pièce, intégralement à l'extrémité distale (10) de l'élément de tige (1).

13. Système obturateur selon une des revendications de 1 à 12, **caractérisé en ce que** l'on prévoit à l'intérieur de la lumière (13) s'étandant d'un bout à l'autre de l'élément de tige (1) une masse abseurbatrice.

14. Système obturateur selon une des revendications de 1 à 13, **caractérisé en ce que** la conduite de remplissage (3) est formée avec une valve et un ballon-sonde (30) afin de reconnaître la pression de remplissage du ballonnet (2).

15. Système obturateur selon une des revendications de 1 à 14, **caractérisé en ce que** l'élément de tige (1) présente un diamètre extérieur allant de 4 à 8 mm et la déformation du ballonnet (20) formant le ballonnet intrarectal présente, dans un état rempli, pas dilaté, un diamètre de 30 à 80 mm et une longeur de 40 mm à 20 cm.

16. Système obturateur selon une des revendications de 1 à 15, **caractérisé en ce que** l'élément de tige (1) et formé de manière épaissi dans la zone de raccordement vers le ballonnet (2).
